# EUROPEAN PATENT SPECIFICATION

(11) **EP 3 092 965 B1**
(45) Date of publication and mention of the grant of the patent: **18.11.2020**
(21) Application number: 16159882.6
(22) Date of filing: 11.03.2016
(51) Int. Cl.: A61B 17/70

(54) **INSTRUMENT FOR USE WITH A BONE ANCHORING DEVICE IN SPINAL SURGERY AND SYSTEM INCLUDING THE INSTRUMENT AND A BONE ANCHORING DEVICE**
INSTRUMENT ZUR VERWENDUNG MIT EINER KNOCHENVERANKERUNGSVORRICHTUNG IN DER WIRBELSÄULENCHIRURGIE UND SYSTEM MIT DEM INSTRUMENT UND EINER KNOCHENVERANKERUNGSVORRICHTUNG
INSTRUMENT DESTINE A ETRE UTILISE AVEC UN DISPOSITIF D'ANCRAGE OSSEUX DANS LA CHIRURGIE DU RACHIS, SYSTEME COMPRENANT LEDIT INSTRUMENT ET UN TEL DISPOSITIF

(30) Priority: 15.05.2015 EP 15167800
(43) Date of publication of application: 16.11.2016
(73) Proprietor: Biedermann Technologies GmbH & Co. KG, 78166 Donaueschingen (DE)
(72) Inventor: DANDANOPOULOS, Dimosthenis, 78056 VS-Schwenningen (DE); BIEDERMANN, Timo, 78647 Trossingen (DE)
(74) Representative: Prüfer & Partner mbB Patentanwälte · Rechtsanwälte

(56) References cited:
- WO-A2-2010/030916
- DE-U1- 29 606 468
- FR-A1- 2 677 242
- FR-A1- 2 985 166
- US-A1- 2008 045 950
- US-A1- 2012 271 365
- US-A1- 2013 030 445
- US-A1- 2014 163 625
- US-A1- 2014 277 137

## Description

The invention relates to an instrument for use with a bone anchoring device in spinal surgery and to a system including the instrument and a bone anchoring device. The bone anchoring device typically includes a bone anchoring section and a receiving part with a channel for receiving a rod. The instrument includes a holding member configured to hold the receiving part and a rod contacting member that is configured to contact the rod and to move the rod towards a bottom of the channel. A particular field of application is the use of the instrument as a rod approximator in a procedure for correcting spinal deformities.

A spinal rod approximator for seating a stabilizing rod in a rod-receiving portion of a spinal implant is known, for example, from US 8,636,776 B2. The rod approximator includes an elongate member having a grasping member formed on a distal end thereof, and a rod pusher member slidably mated to or mounted on the elongate member. The grasping member is effective to grasp a portion of a spinal implant, and the pusher member is effective to grasp and engage a stabilizing rod and push the rod into a rod-receiving portion of the spinal implant being grasped by the grasping member.

US 2008/0077134 A1 describes a percutaneous access device comprising a hollow elongate member having an inner lumen extending therethrough and opposed arms coupled by at least one fulcrum such that the opposed arms are adapted to pivot relative to one another to releasably engage a bone anchor between a distal end of the opposed arms and a locking mechanism disposed between the opposed arms and movable between an unlocked position in which the opposed arms are free to pivot relative to one another and a locked position in which the locking mechanism prevents the opposed arms from pivoting toward and away from one another to lock a bone anchor into engagement with the opposed arms.

US 2004/0143265 describes a detachable member used as a guide to install bone fasteners of a bone fastener assembly in vertebral bone. The detachable member may comprise a sleeve coupled to a collar of a bone fastener assembly. In some embodiments, the sleeve may comprise a pair of hinged arms and a flange of the sleeve may contact a bottom portion of the collar of the bone fastener.

WO 2010/030916 A2 discloses a reduction tool for use in posterior spinal fixation to facilitate insertion of a longitudinal spinal rod into a rod receiving channel formed in a bone fixation element. The tool comprises an inner tube, an outer tube and a rotatable sleeve coupled to a proximal end of the inner tube and having an external thread engaging an inner thread of the outer tube such that rotation of the sleeve advances the inner tube with respect to the outer tube. The outer tube has a distal end at which an engagement feature for engaging corresponding feature formed on an outer surface of a body portion of the bone fixation element is provided.

US 2013/0030445 A1 discloses a surgical instrumentation for spinal surgery, which includes a downtube having a tubular body having a proximal end for receiving a surgical tool such as a rod persuader and a distal end forming at least two attachment members, or arms, for attaching to a bone anchor. Each arm has a bearing surface facing an inner passage, and engagement elements in the form of fixed circular tabs projecting into the passage for attachment to the bone anchor are formed on the bearing surface. A saddle-shaped head of the bone anchor has corresponding recesses which cooperate with the tabs to allow establishing a secure connection. US 2014/0163625 A1 discloses an instrument for reducing a spinal rod within a screw extender that attaches to a spinal implant.

It is the object of the invention to provide an improved instrument for use with a bone anchoring device in spinal surgery and a system including the instrument and a bone anchoring device that is simple to handle and can be easily assembled and disassembled.

The object is solved by an instrument according to claim 1 and by a system of an instrument and a bone anchoring device according to claim 14. Further developments are given in the dependent claims.

With the instrument, the receiving part of a bone anchoring device that has been implanted into a vertebra, in particular into the pedicle, can be easily located and the holding member of the instrument can be easily connected to the receiving part. Because the holding member engages the receiving part at a bottom end of the receiving part, particular engagement portions, such as notches, formed in an outer wall of the receiving part for engagement with the instrument are not necessary. Therefore, the instrument can be used together with a great variety of existing receiving parts of bone anchors.

The instrument comprises few parts and can be quickly assembled for use and disassembled for cleaning and sterilization purposes. In an embodiment, the holding member is monolithic and the arms of the holding member are connected through a spring portion. The spring function can be obtained by a specific shape of the connection portion, such as an arc-shape. This design contributes to the reduction of parts of the instrument and provides a good holding or retention force for holding the receiving part in the holding member.

In another embodiment, the arms, more in particular, the engagement portion comprises a recess or protrusion or another engagement structure for a form fit-engagement with a protrusion or recess or other complementary engagement structure, respectively at the bottom of the receiving part. This enhances the safety of the retention of the receiving part in the holding member.

An actuating member for actuating the rod contacting member also maintains the instrument in the closed position of the arms, when the receiving part is held between the arms of the holding member. Hence, an inadvertent spreading of the arms in the closed position cannot not occur. The rod contacting member is held in the actuating member and can be advanced to press onto the rod. After seating of the rod, a locking element for locking the rod in the receiving part can be passed through the rod contacting member and tightened to fix the rod. This procedure can be performed quickly due to the simple design.

The instrument may be used in particular as a spinal rod approximator for pulling a middle vertebra out of three adjacent vertebra towards the rod in a procedure where at least three bone anchoring devices are used to correct a spinal deformity. A particular suitable field of application is minimally invasive surgery and minimal access surgery, where only small incisions are made and where it is important to locate the implanted bone anchors easily and quickly with the instrument.

Further features and advantages of the invention will become apparent from the description of embodiments by means of the accompanying drawings. In the drawings:
- Fig. 1: shows a perspective view of an embodiment of the instrument together with a spinal stabilization system comprising bone anchoring elements inserted into adjacent vertebrae;
- Fig. 2: shows a perspective exploded view of the instrument shown in Fig. 1;
- Fig. 3: shows a cross-sectional view of the assembled instrument of Fig. 2 with the crosssection taken in a plane perpendicular to a plane of symmetry and including a longitudinal central axis of the instrument.
- Fig. 4: shows a perspective view from above of a holding member of the instrument of Figs. 1 to 3.
- Fig. 5: shows a perspective view from the bottom of the holding member of Fig. 4.
- Fig. 6: shows a top view of the holding member of Figs. 4 and 5.
- Fig. 7: shows a cross-sectional view of the holding member of Figs. 4 and 5 along line A-A in Fig. 6.
- Fig. 8: shows a perspective view from above of a rod contacting member of the instrument of Figs. 1 to 3.
- Fig. 9: shows a perspective view from the bottom of the rod contacting member of Fig. 8.
- Fig. 10: shows a top view of the rod contacting member of Figs. 8 and 9.
- Fig. 11: shows a cross-sectional view of the rod contacting member of Figs. 8 and 9 along line B-B in Fig. 10.
- Fig. 12: shows a perspective view from above of an actuating member of the instrument of Figs. 1 to 3.
- Fig. 13: shows a perspective view from the bottom of the actuating member shown in Fig. 12.
- Fig. 14: shows a top view of the actuating member of Figs. 12 and 13.
- Fig. 15: shows a cross-sectional view of the actuating member of Figs. 12 and 13 along line D-D in Fig. 14.
- Figs. 16a to 16e: show cross-sectional views of steps of use of the instrument during correction of a spinal deformity.
- Fig. 17a: shows a perspective view of a step of locking a bone anchoring device after the step shown in Fig. 16e.
- Fig. 17b: shows an enlarged view of a detail of Fig. 17a.
- Fig. 18: shows a perspective view of a lower end portion of the holding member of the instrument according to a further embodiment.
- Fig. 19: shows a perspective view from the bottom of a receiving part that is configured to cooperate with the holding member according to Fig. 18.
- Fig. 20: shows a cross-sectional view of a portion of the instrument according to the further embodiment together with a receiving part according to Fig. 19.
- Fig. 21: shows a perspective view of a lower end portion of a holding member according to a still further embodiment.
- Fig. 22: shows a perspective view from the bottom of a receiving part that is configured to cooperate with the holding member of Fig. 21.
- Fig. 23: shows a cross-sectional view of a portion of the holding member of Fig. 21 together with a receiving part of Fig. 22.

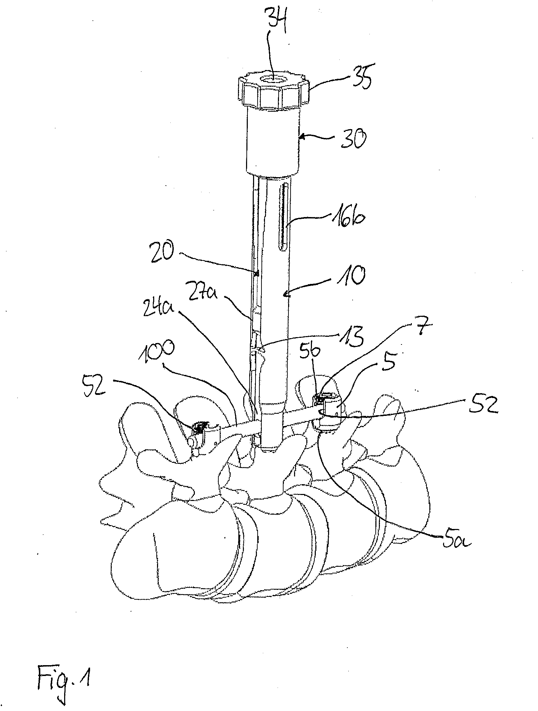

Referring to Figs. 1 to 3, the instrument includes a holding member 10 that is adapted to be attached to a receiving part 5 of a bone anchoring device and to hold the receiving part 5. Further, the instrument includes a rod contacting member 20 that is adapted to contact a rod 100 that is inserted into the receiving part 5 and connects the receiving parts 5 of a plurality of bone anchoring devices. Each receiving part has a bottom end 5a facing the bone surface and an opposite top end 5b. The rod contacting member 20 is connectable to the holding member 10 via an actuating member 30. The actuating member 30 is also configured to move the rod contacting member 20 such that the rod 100 can be advanced into a channel 52 of the receiving part 5.

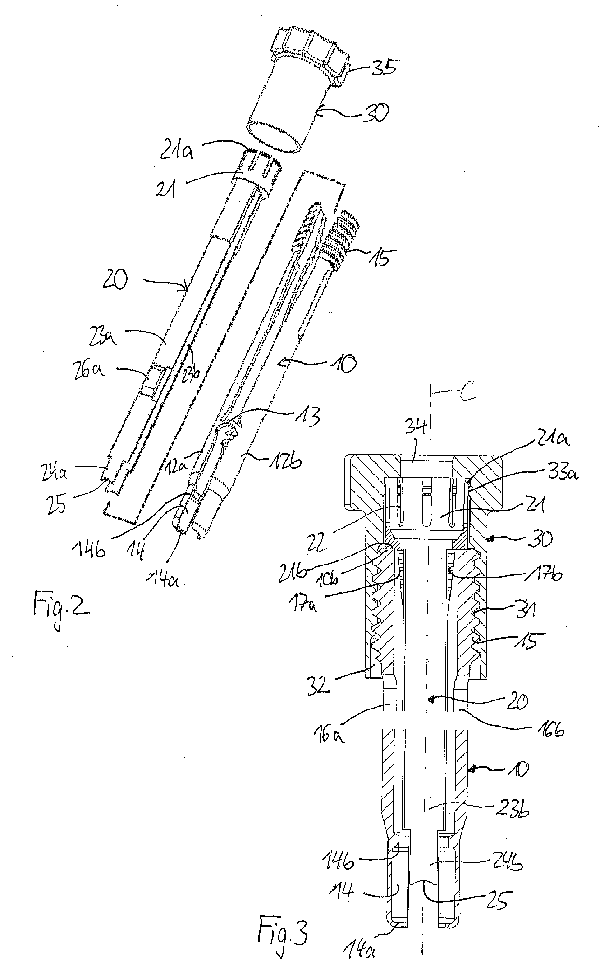

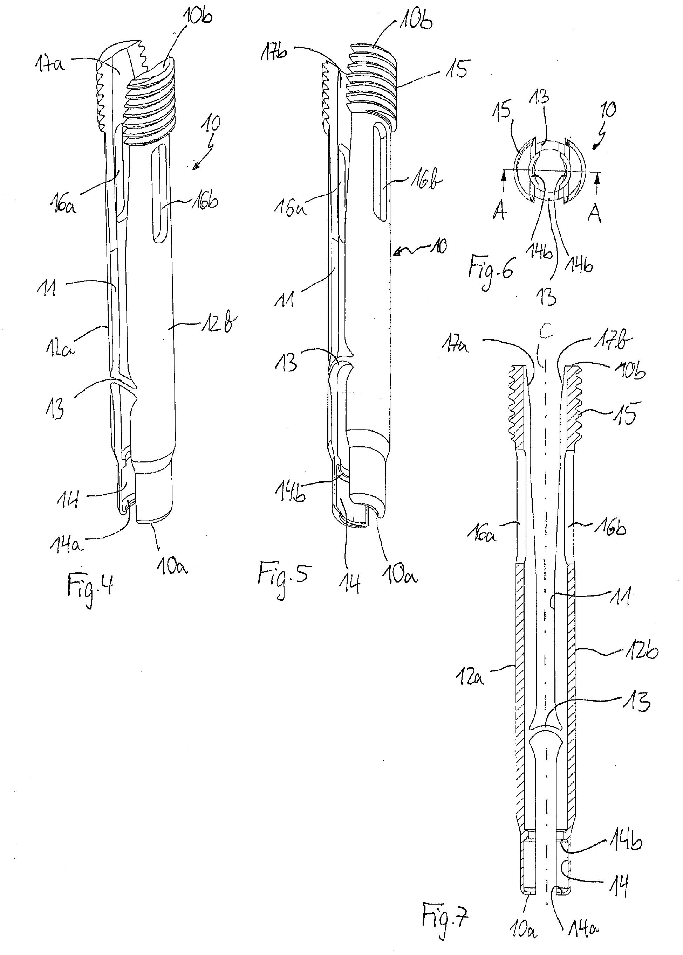

Referring more in detail to Figs. 4 to 7, the holding member 10 is an elongate member having a first end 10a and an opposite second end 10b. It is a substantially tubular member with a substantially cylindrical outer surface, a central longitudinal axis C and a slot 11 extending from the first end 10a to the second end 10b. By means of the slot 11 two arms 12a, 12b are formed. The distance between the arms, i.e. the width of the slot 11, has a size such that the rod contacting member 20 can be guided therethrough as shown in Fig. 3. At a distance of the first end 10a a connecting portion 13 is provided at both sides of the slot 11 (only one side is shown) that connects the arms 12a, 12b together. The distance of the connecting portion 13 from the first end 10a may be smaller than the distance of the connecting portion 13 from the second end 10b. The connecting portion 13 has the shape of an arcuate bridge with a curvature that is concave when seen from the first end 10a. The thickness of the connecting portion 13 and the curvature is selected such that the connecting portion 13 acts as a spring between the arms 12a, 12b. More specifically, the connecting portion 13 forms a fulcrum that permits the arms 12a, 12b of the holding member 10 to perform a pivoting movement like pliers. Moving the arms 12a, 12b towards each other at the second end 10b results spreading the arms 12a, 12b at the first end 10a. Thereby an open position of the holding member 10 is defined in which a receiving part can be inserted between the arms 12a, 12b. When the arms 12a, 12b are released at the second end 10b, the return force of the spring portion moves the arms 12a, 12b together at the first end 10a whereby a closed position is defined in which the arms 12a, 12b are substantially parallel to each other.

The holding member 10 further comprises an engagement portion 14 at an inner wall of the arms 12a, 12b with an annular projection 14a at the first end 10a and at a distance therefrom an annular projection 14b wherein the distance between the annular projections 14a, 14b corresponds substantially to an axial height, i.e. a distance between the bottom end 5a and the top end 5b of a receiving part 5 to be engaged. An inner contour of the engagement portion 14 corresponds substantially to an outer contour of the receiving part 5 to be engaged therewith. The size of the engagement portion 14 is such that in the closed position the receiving part 5 can be held between the projection 14a at the first end 10a and the upper projection 14b in a positive fit manner so that an axial displacement of the receiving part is prevented.

Adjacent to the second end 10b an advancement structure in the form of an external thread 15 is provided on the arms 12a, 12b. The external thread 15 is configured to engage a corresponding thread in the actuating member 30. As depicted in Fig. 7, in the neutral position of the arms 12a, 12b wherein the arms 12a, 12b are substantially parallel, the actuating member 30 can be screwed onto the holding member 10 without jamming.

In the vicinity of the external thread 15 two elongate windows 16a, 16b, may be provided in the wall of the arms 12a, 12b. The windows 16a, 16b may serve for facilitating cleaning of the instrument. It shall be noted that an outer diameter of the holding member 10 may be reduced in the region of the engagement portion 14. This may be of advantage if the receiving parts are placed very close together. Adjacent to the second end 10b, each arm 12a, 12b comprises an inclined inner surface portion 17a, 17b that provides an increased inner diameter towards the second end 10b for facilitating the insertion of the rod contacting member 20.

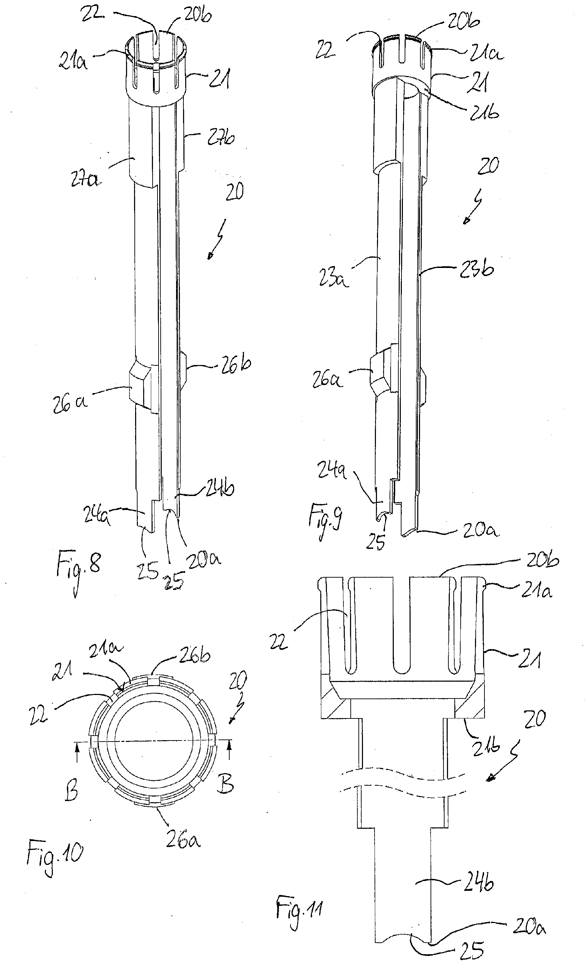

Referring now more in detail to Figs. 8 to 11, the rod contacting member 20 is a substantially tubular member having a first end 20a and an opposite second end 20b. A tubular connecting portion 21 adjacent to the second end 20b is provided that has a plurality of slits 22 open to the second end 20b for rendering the tubular connecting portion 21 flexible in a radial direction. The tubular connecting portion 21 has a greater external diameter than the external diameter of the holding member 10 and serves for a snap-in connection with the actuating member 30 as described below. For that purpose at the free end of the tubular connecting portion a small collar or thickened end portion 21a is provided that is configured to snap behind a protrusion in the actuating member 30. A bottom end 21b of the tubular connecting portion 21 provides a stop for the second end 10b of the holding member 10 (see Fig. 3).

From the tubular connecting portion 21 two arms 23a, 23b extend up to the first end 20a. The arms 23a, 23b have substantially cylindrical inner and outer surfaces. A basic outer diameter of the arms 23a, 23b is such that the rod contacting member 20 fits in-between the arms 12a, 12b of the holding member 10 and is guided therebetween. An inner diameter of the rod contacting member 20 between the arms 23a, 23b is such that a locking element for locking the rod in the receiving part can be passed therethrough. An inner diameter of the tubular connecting portion 21 may be greater than an inner diameter between the arms 23a, 23b in order to provide sufficient space for compressing the flexible wall portions during insertion into the actuating member 30.

Adjacent to the first end 20a, each of the arms 23a, 23b comprises a portion 24a, 24b, respectively, with a reduced width in circumferential direction and with a rod contacting surface 25 that is adapted to contact the rod 100. The rod contacting surface 25 may be cylindrical and adapted to the cylindrical shape of the rod 100. However, it can also be substantially V-shaped to permit contacting rods with different diameters. At an outer surface of the arms 23a, 23b guiding portions 26a, 26b, respectively, with a greater outer diameter than the basic outer diameter of the arms 23a, 23b are provided. A width of the guiding portions 26a, 26b in a circumferential direction is slightly smaller than a width of the slot 11 of the holding member 10. By the provision of the guiding portions 26a, 26b, the rod contacting member 10 can be inserted into the holding member 10 only in a position in which the guiding portions 26a, 26b are aligned with the slot 11. In this orientation, the rod contacting surface 25 is aligned with the channel 52 in the receiving part and can contact a rod 100 placed therein. Additional guiding portions 27a, 27b with a slightly increased outer diameter compared to the basic diameter of the arms 23a, 23b may be provided adjacent to the tubular portion 21 for additional guidance in the upper portion of the slot 11 of the holding member 10.

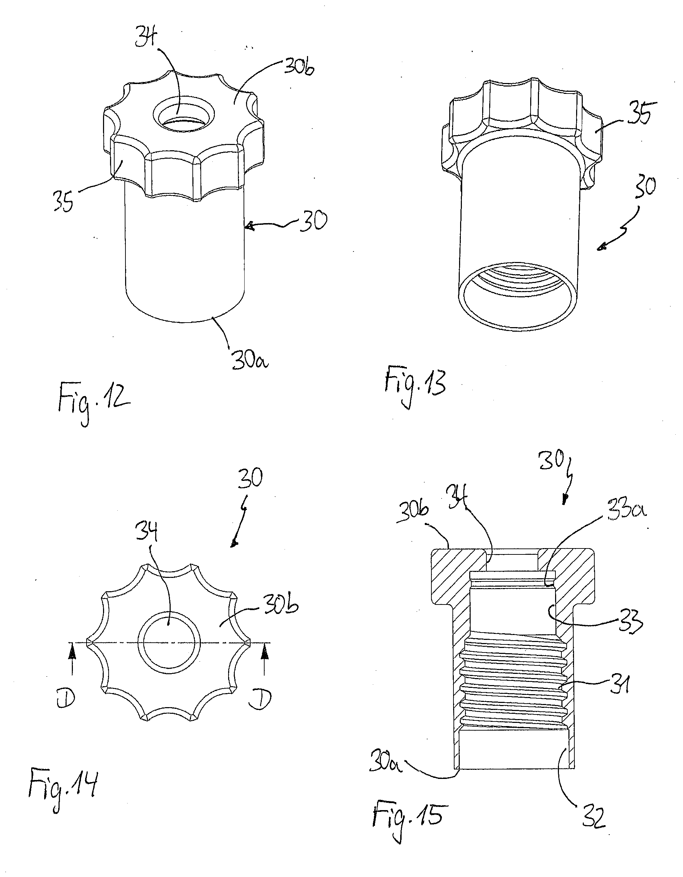

Turning now to Figs. 12 to 15, the actuating member 30 is a substantially tubular member with a first end 30a and an opposite second end 30b and an internally threaded portion 31 between the first end 30a and the second end 30b, wherein the threaded portion 31 cooperates with the external thread 15 of the holding member. Adjacent to the first end 30a, a section 32 with a slightly greater inner diameter compared to the threaded section 31 may be provided that facilitates mounting onto the tubular connecting portion 21 of the rod contacting member 20. An inner diameter of the threaded portion 31 is greater than an outer diameter of the tubular portion 21 of the rod contacting member 20 such that the tubular portion can pass through the internally threaded portion 31.

Between the internally threaded portion 31 and the second end 30b, an accommodation section 33 is formed that is configured to receive the tubular connecting portion 21 of the rod contacting portion 20. At a distance from the second end 30b an annular protrusion 33a is provided in the accommodation section 33 that forms together with the collar 21a of the tubular connecting portion 21 a holding structure for holding the rod contacting member 20 and the actuating member 30 together. The tubular portion 21 of the rod contacting member 20 is slightly compressed when inserted into the accommodation section 33 and when the collar 21a snaps behind the annular protrusion 33a so that the rod contacting member 20 is held in the actuating member 30. In the mounted state, the rod contacting member 20 is able to rotate with respect to the actuating member 30 in the mounted state. A passage 34 between the second end 30b and the accommodation section 33 has a size so as to permit the insertion of a locking element therethrough. Additionally, the actuating member 30 comprises at or near the second end a grip portion 35 at its outer surface with a structure to permit gripping and rotating the actuating member by hand.

The parts of the instrument can be made of a bio-compatible material, such as a bio-compatible metal or a bio-compatible metal alloy, for example stainless steel, titanium, NiTi-alloys, such as Nitinol, magnesium or magnesium alloys or from a bio-compatible plastic material, such as, for example, polyetheretherketone (PEEK) or poly-1-lactide acid (PLLA). The parts can be made of the same or of different materials. It should be noted that a NiTi-alloy having super-elastic properties may be used to achieve a spring effect for the connecting portion 13 of the holding member 10.

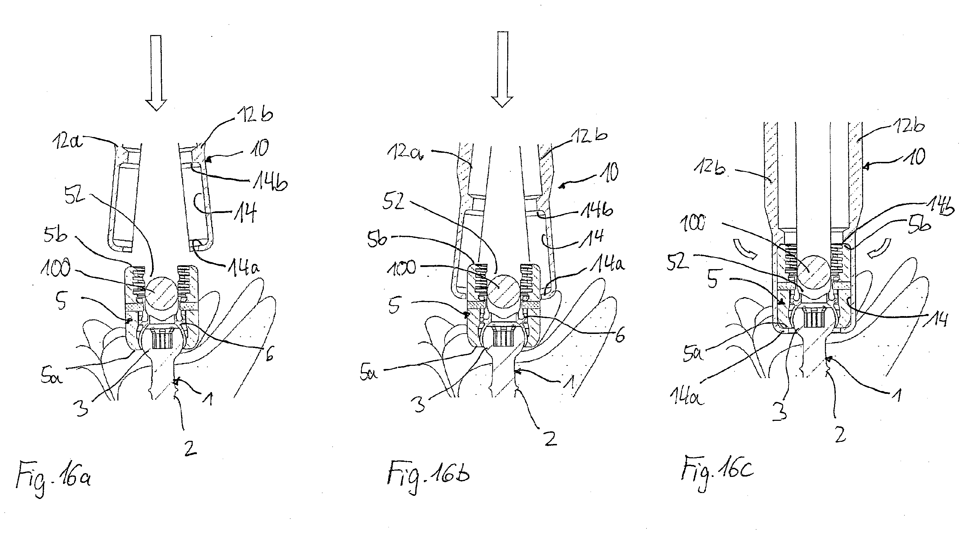

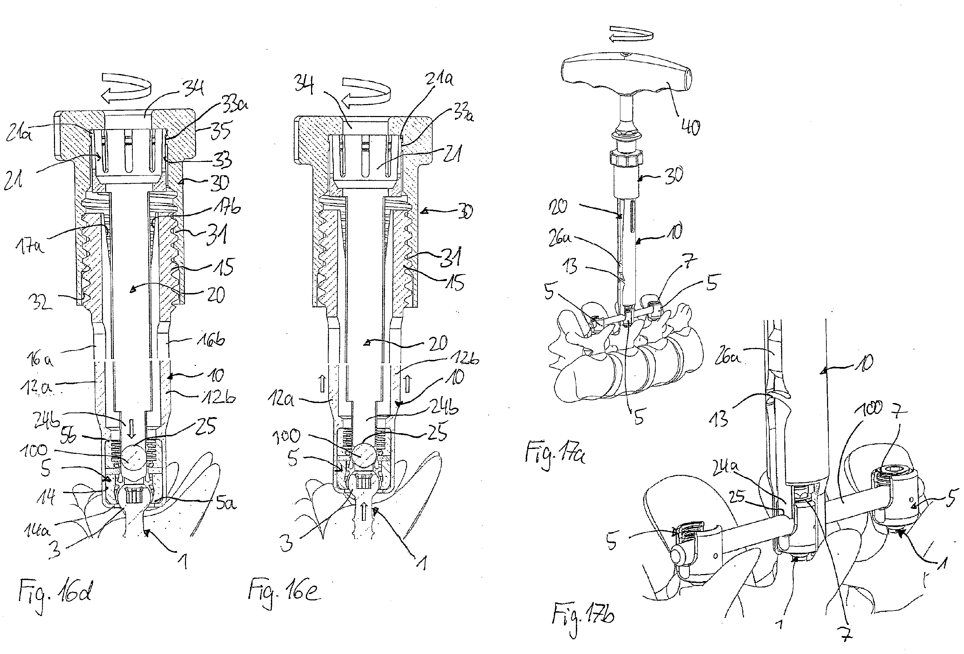

Referring now to Figs. 16a to 17b, the use of the instrument will be explained by means of an exemplary correction of a spinal deformity. First, when using minimally invasive surgery, a small incision is made in the skin of the patient and then a plurality of bone anchoring devices, for example three bone anchoring devices, are implanted in the pedicles on each side of adjacent vertebrae (only one side is shown in Figs.1, 17a and 17b). The bone anchoring devices may be polyaxial bone anchoring devices with an anchoring element 1 with a shank 2 to be connected to the bone and a head 3 that is pivotably held in the receiving part 5 and can be locked at an angle relative to the receiving part via a pressure element 6 (Figs. 16a to 16e). The receiving parts 5 are pivoted until they are aligned and the rod 100 is inserted into the channel.

In one of the outer receiving parts 5 a locking element 7, for example a set screw, is inserted to fix the rod 100 and the head of the bone anchoring element 1 in the receiving part 5. Usually, the rod 100 is seated in the bottom of the channel of the two outer receiving parts 5. Between the bottom of the channel of the middle receiving part and the rod there may be a gap and the middle receiving part is pulled towards the rod 100 using the instrument as described in the following.

As illustrated in Fig. 16a, first, the holding member 10 is moved towards the receiving part 5. At this stage, the holding member is not assembled with the rod contacting member 20. Therefore, the arms 12a, 12b can be manually compressed towards each other at the second end 10b. This causes the arms 12a, 12b to spread apart at the first end 10a as depicted in Fig. 16a to assume the open position. In the open position, the holding member 10 can be placed onto the receiving part as illustrated in Fig. 16b. Correct alignment of the instrument is guaranteed by the inserted rod 100 that has to enter in-between the arms and by the guidance of the rod contacting member 20 in the holding member 10. When the holding member 10 is moved further downwards, the first end 10a of the arms 12a, 12b slides along the outer surface of the receiving part 5 until it slides under the bottom end 5a of the receiving part. Due to the biasing force of the connecting portion 13, the arms 12a, 12b return to their closed position as shown in Fig. 16c when the arms 12a, 12b are released at the second end 10b. In the closed position, the engagement portion 14 engages the receiving part from the bottom end to the top end 5b, wherein the inner projections 14a, 14b hold the receiving part 5 in a positive-fit member in an axial direction so that the holding member 10 can not slip off.

Thereafter, as depicted in Fig. 16d, the actuating member 30 that is preassembled with the rod contacting member 20 is inserted in-between the arms 12a, 12b of the holding member 10 in such an orientation that the guiding portions 26a, 26b enter into the slot 11 and guide the rod contacting member 20 therein. The actuating member 30 is screwed onto the holding member 10 until the rod contacting surface 25 of the rod contacting member 20 contacts the inserted rod 100 as shown in Fig. 16d. Next, as depicted in Fig. 16e, further downward screwing of the actuating member 30 pulls the receiving part 5 that is held by the holding member 10 upward towards the rod 100. Because the rod contacting member 20 is rotatably held in the actuating member 30, it can function as a counter-holding portion for the upward movement of the receiving part 5. The vertebra moves together with the receiving part 5 slightly upward, until the rod 100 is seated in the bottom of the channel 52. Finally, as depicted in Figs. 17a and 17b, a locking element 7 is placed with a tool 40 through the instrument into the receiving part 5 and tightened to lock the rod 100 and the receiving part 5 with respect to each other and also to lock the angular position of the bone anchoring element 1 with respect to the receiving part 5.

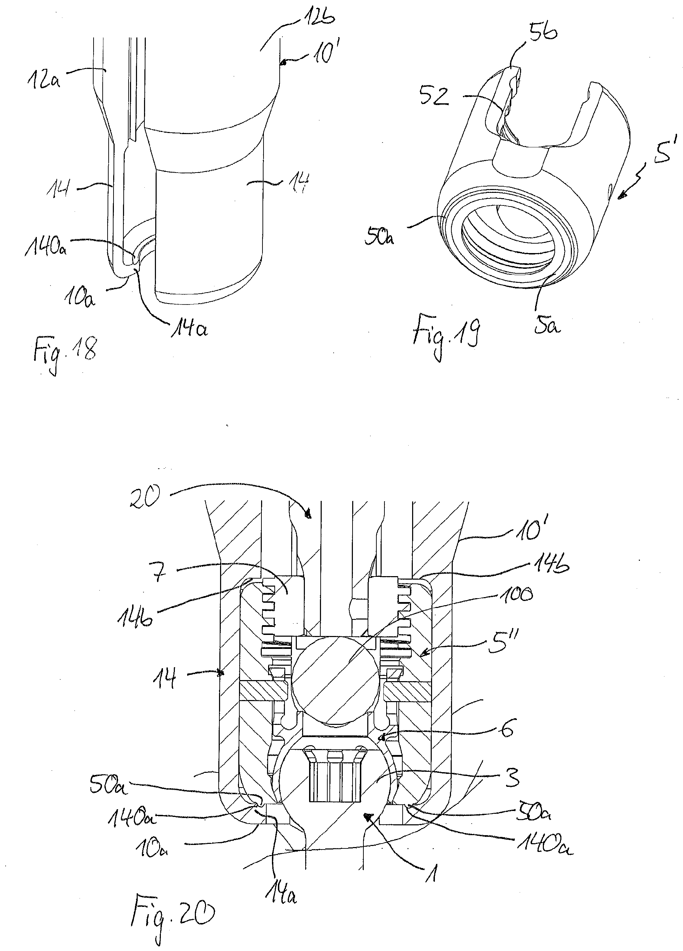

Referring to Fig. 18, a further embodiment of the instrument comprises a holding member 10' that differs from the holding member of the previous embodiment. Parts and portions of the instrument that are identical or highly similar to that of the previous embodiment are indicated with the same reference numerals and the description thereof will not be repeated. The holding member 10' comprises an annular groove 140a at an inner wall of the annular projection 14a at the first end 10a. More in detail, the annular groove 140a is arranged such that it faces in a direction towards the second end 10b of the holding member. The annular groove 140a extends in a circumferential direction along the whole annular projection 14a. A cross-section of the annular groove 140a may be circular segment-shaped. However, any cross-section that allows an engagement with a corresponding portion of the receiving part may be also selected for the groove 140a. Preferably, however, the groove 140a has a rounded contour. The groove 140a may be shallow so as to allow an easy engagement with the receiving part.

As shown in Fig. 19, a receiving part 5' that is configured to be engaged with the instrument differs from the receiving part 5 of the previous embodiment, that it comprises a circular protrusion 50a at the bottom end 5a that is at such a distance from the central axis C that it can engage the annular groove 140a of the holding member 10'. Also the shape and size of the protrusion 50a is such that the protrusion 50a fits into the annular groove 140a of the holding member 10'. As shown in Fig. 19, the protrusion extends completely around the central axis at the bottom end 5a, however, it may also be sufficient, that the protrusion is only at the sides of the channel 52 for receiving the rod.

In use, the holding member is brought into the open position, where the arms 12a, 12b are spread apart at the first end 10a and placed onto the receiving part 5' until the arms 12a, 12b slide under the bottom end 5a of the receiving part 5'. When the arms 12a, 12b return to their closed position as shown in Fig. 20, the annular protrusion 50a at the bottom end 50a of the receiving part 5' snaps into the annular groove 140a of the holding member 10'. The engagement of the annular protrusion 50a and the annular groove 140a increases the holding force exerted by the holding member 10'. In the configuration shown in Fig. 20, the rod contacting member 20 holds the locking member 7 and can tighten it to fix the rod.

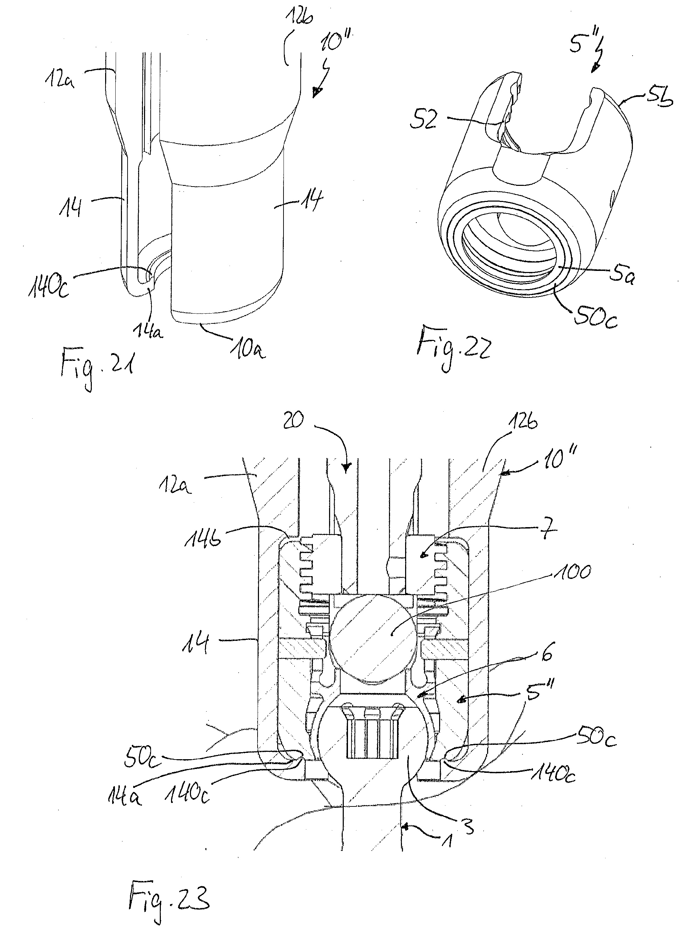

In a still further embodiment as depicted in Figs. 21 to 23, a holding member 10" comprises an annular protrusion 140c of the annular projection 14a. The receiving part 5" comprises a complementary annular groove 50c at the bottom end 5a that is configured to cooperate with the annular protrusion 140c. The use and effect of the cooperating grooves and protrusions is the same as for the previous embodiment. Fig. 23 shows the engagement of the annular protrusion 140c at the holding member 10" in the annular groove 50c of the receiving part.

A system including the instrument as described before and a receiving part comprises a holding member 10, 10' that has an engagement portion 14, 14' for engaging the receiving part, wherein the engagement portion is configured to engage the receiving part 5, 5' at the bottom end 5a. It may be further configured to encompass the receiving part 5, 5' from the bottom end 5a to the top end 5b.

Modifications of the above-described embodiments are conceivable. For the bone anchoring device all kinds of bone anchoring devices can be used, such as monoaxial screws, where the anchoring section and the receiving part are fixedly connected to each other, polyaxial bone anchoring devices of the top-loading type, where the bone anchoring element is mountable from the top end into the receiving part, or of the bottom-loading type, where the bone anchoring element is mountable from the bottom end of the receiving part or any other bone anchoring devices that are connectable to a rod.

The connection between the rod contacting member 20 and the holding member 10 can be realized in other ways. For example, the tubular connecting portion 21 may be solid, without exhibiting flexibility, and one or more flexible members inside the actuating member 30 may snap into the tubular connecting portion.

The shape of the connecting portion 13 of the two arms 12a, 12b of the holding member may be different. The annular projections 14a, 14b at the holding member that engage the receiving part 5 need not to be fully annular, any portions that can engage and hold the receiving part may be sufficient.

## Claims

1. Instrument for use with a bone anchoring device in spinal surgery,
wherein the bone anchoring device (1) includes a bone anchoring section (2) and a receiving part (5) having a bottom end (5a) adjacent to the bone anchoring section, a top end (5b) opposite to the bottom end (5a) and channel (52) for receiving a rod (100), the channel (52) having a bottom for supporting the rod, the instrument including
a holding member (10, 10', 10") that is configured to hold the receiving part (5),
wherein the holding member (10, 10', 10") comprises a first arm (12a) and a second arm (12b) that are movable relative to each other between an open position in which the receiving part (5) is insertable between the first arm (12a) and the second arm (12b) into a closed position in which the receiving part (5) is held between the first arm (12a) and the second arm (12b), and
a rod contacting member (20) that is configured to contact the rod (100),
wherein the rod contacting member (20) is movable between the first arm (12a) and the second arm (12b) such that in the closed position of the first arm (12a) and second arm (12b) the rod contacting member can be advanced to move the rod towards the bottom of the channel; **characterized in that** the holding member (10) comprises an engagement portion (14) at each of the arms (12a, 12b) that is adapted to engage the receiving part (5) at the bottom end (5a).

2. The instrument of claim 1 wherein the engagement portion (14) comprises a lower projection (14a) and an upper projection (14b) that have a distance from each other that mates a distance between the bottom end (5a) and the top end (5b) of the receiving part (5, 5', 5") so that the engagement portion (14) engages the receiving part (5, 5', 5") at the bottom end (5b) and at the top end (5a).

3. The instrument of claim 1 or 2, wherein the first arm (12a) and the second arm (12b) each have a first end (10a) and a second end (10b) and wherein the arms (12a, 12b) are connected to each other at a position between the first end (10a) and the second end (10b) in a pliers-like manner such that moving the second ends (10b) towards each other moves the first ends (10a) away from each other.

4. The instrument of claim 3, wherein the first arm (12a) and the second arm (12b) are connected via a spring portion (13).

5. The instrument of one of claims 1 to 4, wherein in the closed position the arms (12a, 12b) are parallel.

6. The instrument of one of claims 1 to 5, wherein the holding member (10, 10', 10") is a monolithic piece.

7. The instrument of one of claims 1 to 6, further comprising an actuating member (30) for actuating the rod contacting member (20), wherein the actuating member (30) is connectable to the holding member (10, 10', 10") and connectable to the rod contacting member (20).

8. The instrument of claim 7, wherein, when the actuating member (30) is connected to the holding member and the rod contacting member (20 is held in the actuating member (30), the actuating member (30) holds the arms (12a, 12b) in the closed position.

9. The instrument of claim 7 or 8, wherein the first arm (12a) and the second arm (12b) comprise an external advancement structure (15) on an outer surface portion and wherein the actuating member (30) comprises an internal advancement structure (31) cooperating with the external advancement structure (15) such that by rotating the actuating member (30) the actuating member (30) is displaced along the arms (12a, 12b), and wherein the advancement structure is preferably a thread.

10. The instrument of one of claims 7 to 9, wherein the rod contacting member (20) is rotatably connectable to the actuating member (30) such that when the actuating member (30) is rotated the rod contacting member (20) is displaced relative to the arms (12a, 12b).

11. The instrument of claim 10, wherein the connection between the rod contacting member (20) and the actuating member (30) is a snap connection.

12. The instrument of one of claims 1 to 11, wherein the rod contacting member (20) is a slotted sleeve a first end (20a) of which comprises a rod contacting surface (25).

13. The instrument of one of claims 1 to 12, wherein the instrument comprises a longitudinal passage for passing through a locking element (7) for locking the rod in the receiving part.

14. A system of a bone anchoring device, wherein the bone anchoring device includes:
a bone anchoring section (2) and a receiving part (5, 5', 5") having a bottom end (5a) adjacent to the bone anchoring section, a top end (5b) opposite to the bottom end (5a) and channel (52) for receiving a rod (100), the channel (52) having a bottom for supporting the rod; and
an instrument according to one of claims 1 to 13.

15. The system of claim 14, wherein the receiving part (5"), comprises at the bottom end (5a) an engagement structure, such as a groove (50c), or a protrusion (50a) that is configured to cooperate with a complementary engagement structure (140c, 50c), at the holding member (10', 10").

## Patentansprüche

1. Instrument zur Verwendung mit einer Knochenverankerungsvorrichtung in der Wirbelsäulenchirurgie,
wobei die Knochenverankerungsvorrichtung (1) einen Knochenverankerungsabschnitt (2) und ein Aufnahmeteil (5) mit einem unteren Ende (5a) benachbart zu dem Knochenverankerungsabschnitt, ein dem unteren Ende (5a) gegenüberliegendes oberes Ende (5b) und einen Kanal (52) zur Aufnahme eines Stabs (100) aufweist, wobei der Kanal (52) einen Boden zum Halten des Stabs besitzt, wobei das Instrument aufweist:
ein Halteelement (10, 10', 10"), das eingerichtet ist, das Aufnahmeteil (5) zu halten,
wobei das Halteelement (10, 10', 10") einen ersten Arm (12a) und einen zweiten Arm (12b) umfasst, die relativ zueinander zwischen einer geöffneten Position, in welcher das Aufnahmeteil (5) zwischen dem ersten Arm (12a) und dem zweiten Arm (12b) einführbar ist, und einer geschlossenen Position, in welcher das Aufnahmeteil (5) zwischen dem ersten Arm (12a) und dem zweiten Arm (12b) gehalten wird, und
ein den Stab kontaktierendes Element (20) das eingerichtet ist, den Stab (100) zu kontaktieren,
wobei das den Stab kontaktierende Element (20) zwischen dem ersten Arm (12a) und dem zweiten Arm (12b) bewegbar ist, so dass das den Stab kontaktierende Element in der geschlossen Position des ersten Arms (12a) und des zweiten Arms (12b) vorgetrieben werden kann, um den Stab in Richtung zum Boden des Kanals zu bewegen; **dadurch gekennzeichnet, dass** das Halteelement (10) einen Eingriffsabschnitt (14) an jedem der Arme (12a, 12b) umfasst, der da-rauf angepasst ist, das Aufnahmeteil (5) an dem unteren Ende (5a) anzugreifen.

2. Das Instrument gemäß Anspruch 1, wobei der Eingriffsabschnitt (14) einen unteren Vorsprung (14a) und einen oberen Vorsprung (14b) umfasst, die einen Abstand voneinander besitzen, der einem Abstand zwischen dem unteren Ende (5a) und dem oberen Ende (5b) des Aufnahmeteils (5, 5', 5") entspricht, so dass der Eingriffsabschnitt (14) das Aufnahmeteil (5, 5', 5") an dem unteren Ende (5b) und an dem oberen Ende (5a) angreift.

3. Das Instrument gemäß Anspruch 1 oder 2, wobei der erste Arm (12a) und der zweite Arm (12b) jeweils ein erstes Ende (10a) und ein zweites Ende (10b) besitzen, wobei die Arme (12a, 12b) miteinander an einer Position zwischen dem ersten Ende (10a) und dem zweiten Ende (10b) in zangenartiger Weise verbunden sind, so dass eine Bewegung der zweiten Enden (10b) in Richtung aufeinander zu die ersten Enden (10a) voneinander wegbewegt.

4. Das Instrument gemäß Anspruch 3, wobei der erste Arm (12a) und der zweite Arm (12b) miteinander über einen Federabschnitt (13) verbunden sind.

5. Das Instrument gemäß einem der Ansprüche 1 bis 4, wobei die Arme (12a, 12b) in der geschlossenen Position parallel sind.

6. Das Instrument gemäß einem der Ansprüche 1 bis 5, wobei das Halteelement (10, 10', 10") ein einstückiges Teil ist.

7. Das Instrument gemäß einem der Ansprüche 1 bis 6, ferner umfassend ein Betätigungselement (30) zum Betätigen des den Stab kontaktierenden Elements (20), wobei das Betätigungselement (30) mit dem Halteelement (10, 10', 10") verbindbar ist und auch mit dem den Stab kontaktierenden Element (20) verbindbar ist.

8. Das Instrument gemäß Anspruch 7, wobei, wenn das Betätigungselement (30) mit dem Halteelement verbunden ist und das den Stab kontaktierende Element (20) in dem Betätigungselement (30) gehalten ist, das Betätigungselement (30) die Arme (12a, 12b) in der geschlossenen Position hält.

9. Das Instrument gemäß Anspruch 7 oder 8, wobei der erste Arm (12a) und der zweite Arm (12b) eine externe Vortriebsstruktur (15) auf einem äußeren Oberflächenabschnitt umfassen, und wobei das Betätigungselement (30) eine interne Vortriebsstruktur (31) umfasst, die mit der externen Vortriebsstruktur (15) so zusammenwirkt, dass durch Drehen des Betätigungselements (30) das Betätigungselement (30) entlang den Armen (12a, 12b) verschoben wird, und wobei die Vortriebsstruktur vorzugsweise ein Gewinde ist.

10. Das Instrument gemäß einem der Ansprüche 7 bis 9, wobei das den Stab kontaktierende Element (20) drehbar mit dem Betätigungselement (30) verbindbar ist, so dass, wenn das Betätigungselement (30) gedreht wird, das den Stab kontaktierende Element (20) relativ zu den Armen (12a, 12b) verschoben wird.

11. Das Instrument gemäß Anspruch 10, wobei die Verbindung zwischen dem den Stab kontaktierenden Element (20) und dem Betätigungselement (30) eine Schnappverbindung ist.

12. Das Instrument gemäß einem der Ansprüche 1 bis 11, wobei das den Stab kontaktierende Element (20) eine geschlitzte Hülse ist, deren erstes Ende (20a) eine den Stab kontaktierende Oberfläche (25) besitzt.

13. Das Instrument gemäß einem der Ansprüche 1 bis 12, wobei das Instrument eine Längspassage zum Durchführen eines Verriegelungselements (7) ist, um den Stab in dem Aufnahmeteil zu verriegeln.

14. Ein System einer Knochenverankerungsvorrichtung, wobei die Knochenverankerungsvorrichtung aufweist:
einen Knochenverankerungsabschnitt (2) und ein Aufnahmeteil (5, 5', 5") mit einem unteren Ende (5a) benachbart zu dem Knochenverankerungsabschnitt, ein dem unteren Ende (5a) gegenüberliegendes oberes Ende (5b) und einen Kanal (52) zur Aufnahme eines Stabs (100), wobei der Kanal (52) einen Boden zum Halten des Stabs besitzt; und
ein Instrument gemäß einem der Ansprüche 1 bis 13.

15. Das System gemäß Anspruch 14, wobei das Aufnahmeteil (5") an dem unteren Ende (5a) eine Eingriffsstruktur wie etwa eine Nut (50c) oder einen Vorsprung (50a) aufweist, die dazu eingerichtet ist, mit einer komplementären Eingriffsstruktur (140c, 50c) an dem Halteelement (10', 10") zusammenzuwirken.

## Revendications

1. Instrument destiné à être utilisé avec un dispositif d'ancrage osseux en chirurgie vertébrale,
dans lequel le dispositif d'ancrage osseux (1) comprend une section d'ancrage osseux (2) et une partie de réception (5) ayant une extrémité inférieure (5a) adjacente à la section d'ancrage osseux, une extrémité supérieure (5b) opposée à l'extrémité inférieure (5a) et au canal (52) pour recevoir une tige (100), le canal (52) ayant un fond pour supporter la tige, l'instrument comprenant :
un élément de support (10, 10', 10") qui est configuré pour supporter la partie de réception (5),
dans lequel l'élément de support (10, 10', 10") comprend un premier bras (12a) et un second bras (12b) qui sont mobiles l'un par rapport à l'autre entre une position ouverte dans laquelle la partie de réception (5) peut être insérée entre le premier bras (12a) et le second bras (12b) dans une position fermée dans laquelle la partie de réception (5) est maintenue entre le premier bras (12a) et le second bras (12b), et
un élément de contact de tige (20) qui est configuré pour être en contact avec la tige (100),
dans lequel l'élément de contact de tige (20) est mobile entre le premier bras (12a) et le second bras (12b) de sorte que dans la position fermée du premier bras (12a) et du second bras (12b), l'élément de contact de tige peut être avancé pour déplacer la tige vers le fond du canal ; **caractérisé en ce que** l'élément de support (10) comprend une partie de mise en prise (14) au niveau de chacun des bras (12a, 12b) qui est adaptée pour mettre en prise la partie de réception (5) au niveau de l'extrémité inférieure (5a).

2. Instrument selon la revendication 1, dans lequel la partie de mise en prise (14) comprend une saillie inférieure (14a) et une saillie supérieure (14b) qui ont une distance entre eux qui se couple à une distance entre l'extrémité inférieure (5a) et l'extrémité supérieure (5b) de la partie de réception (5, 5', 5") de sorte que la partie de mise en prise (14) met en prise la partie de réception (5, 5', 5") au niveau de l'extrémité inférieure (5b) et au niveau de l'extrémité supérieure (5a).

3. Instrument selon la revendication 1 ou 2, dans lequel le premier bras (12a) et le second bras (12b) ont chacun une première extrémité (10a) et une seconde extrémité (10b) et dans lequel les bras (12a, 12b) sont raccordés entre eux dans une position entre la première extrémité (10a) et la seconde extrémité (10b) à la manière d'une pince, de sorte que le déplacement des secondes extrémités (10b) l'une vers l'autre déplace les premières extrémités (10a) à distance l'une de l'autre.

4. Instrument selon la revendication 3, dans lequel le premier bras (12a) et le second bras (12b) sont raccordés via une partie de ressort (13).

5. Instrument selon l'une des revendications 1 à 4, dans lequel, dans la position fermée, les bras (12a, 12b) sont parallèles.

6. Instrument selon l'une des revendications 1 à 5, dans lequel l'élément de support (10, 10', 10") est une pièce monolithique.

7. Instrument selon l'une des revendications 1 à 6, comprenant en outre un élément d'actionnement (30) pour actionner l'élément de contact de tige (20), dans lequel l'élément d'actionnement (30) peut être raccordé à l'élément de support (10, 10', 10") et peut être raccordé à l'élément de contact de tige (20).

8. Instrument selon la revendication 7, dans lequel, lorsque l'élément d'actionnement (30) est raccordé à l'élément de support et que l'élément de contact de tige (20) est maintenu dans l'élément d'actionnement (30), l'élément d'actionnement (30) maintient les bras (12a, 12b) dans la position fermée.

9. Instrument selon la revendication 7 ou 8, dans lequel le premier bras (12a) et le second bras (12b) comprennent une structure d'avancement externe (15) sur une partie de surface externe et dans lequel l'élément d'actionnement (30) comprend une structure d'avancement interne (31) coopérant avec la structure d'avancement externe (15) de sorte qu'en faisant tourner l'élément d'actionnement (30), l'élément d'actionnement (30) est déplacé le long des bras (12a, 12b) et dans lequel la structure d'avancement est de préférence un filetage.

10. Instrument selon l'une des revendications 7 à 9, dans lequel l'élément de contact de tige (20) peut être raccordé, en rotation, à l'élément d'actionnement (30) de sorte que lorsque l'élément d'actionnement (30) tourne, l'élément de contact de tige (20) est déplacé par rapport aux bras (12a, 12b).

11. Instrument selon la revendication 10, dans lequel le raccordement entre l'élément de contact de tige (20) et l'élément d'actionnement (30) est un raccordement par encliquetage.

12. Instrument selon l'une des revendications 1 à 11, dans lequel l'élément de contact de tige (20) est un manchon fendu, dont une première extrémité (20a) comprend une surface de contact de tige (25).

13. Instrument selon l'une des revendications 1 à 12, dans lequel l'instrument comprend un passage longitudinal pour traverser un élément de verrouillage (7) afin de verrouiller la tige dans la partie de réception.

14. Système d'un dispositif d'ancrage osseux, dans lequel le dispositif d'ancrage osseux comprend :
une section d'ancrage osseux (2) et une partie de réception (5, 5', 5") ayant une extrémité inférieure (5a) adjacente à la section d'ancrage osseux, une extrémité supérieure (5b) opposée à l'extrémité inférieure (5a) et au canal (52) pour recevoir une tige (100), le canal (52) ayant un fond pour supporter la tige ; et
un instrument selon l'une des revendications 1 à 13.

15. Système selon la revendication 14, dans lequel la partie de réception (5") comprend, au niveau de l'extrémité inférieure (5a), une structure de mise en prise telle qu'une rainure (50c) ou une saillie (50a) qui est configurée pour coopérer avec une structure de mise en prise complémentaire (140c, 50c), au niveau de l'élément de support (10', 10").
